# EUROPÄISCHE PATENTANMELDUNG

(11) **EP 1 260 213 A2**
(43) Veröffentlichungstag der Anmeldung: **27.11.2002**
(21) Anmeldenummer: 02010418.8
(22) Anmeldetag: 08.05.2002
(51) Int. Cl.: A61K 9/00, A61K 39/00

(54) **Darreichungsform von immunologischen Wirkstoffen**

(30) Priorität: 17.05.2001 DE 10125731
(71) Anmelder: A.I.D. Autoimmun Diagnostika GmbH, 72479 Strassberg (DE)
(72) Erfinder: Schöllhorn, Dr.Volkmar, 72479 Strassberg (DE)
(74) Vertreter: Patentanwälte Ruff, Wilhelm, Beier, Dauster & Partner

(57) **Zusammenfassung**

Die Erfindung betrifft eine Darreichungsform von immunologischen Wirkstoffen für die orale oder nasale Behandlung und insbesondere Aufnahme durch die Mund- oder Nasenschleimhaut. Dabei liegen die Wirkstoffe als immunologisch wirksame Wirkstoffpaare mit mindestens einem Antigen und mindestens einem Immunsignalstoff zusammen mit mindestens einer Trägersubstanz vor.

## Beschreibung

Die Erfindung betrifft die spezifische Beeinflussung des Immunsystems. Genauer gesagt wird durch Aufnahme mindestens eines Wirkstoffpaares spezifisch das Immunsystem stimuliert oder supprimiert.

Es ist bekannt, daß bestimmte Zusätze, wie z.B. Vitamine oder Spurenelemente, Getränken und Lebensmittel zugeführt werden. So werden beispielsweise vielen Nahrungsmittel, wie Nährpräparate, Margarine und Milch, sowie Getränke mit Vitaminen angereichert. Zur Vermeidung von Mangelerscheinungen aufgrund von Jod- oder Fluormangel (z.B. Schilddrüsenvergrößerung, Karies etc.) erfolgt eine systematische Jod- und Fluorzufuhr in der Lebensmittelproduktion und bei Mundhygieneartikel (Kochsalz, Zahnpasta, Trinkwasser). Bei dieser Form der unspezifischen Zufuhr benötigter Vitamine und Spurenelemente kann aber nicht auf die individuellen Bedürfnisse des Konsumenten eingegangen werden, wie z.B. vorhandene Prädisposition, Erkrankung etc..

Wirkstoffe werden auch bei der Immunisierung (z.B. Impfung) verabreicht. Es wird hierbei durch die Zufuhr eines Antigens oder eines Antigenproduzenten die Abwehrfähigkeit des Immunsystems durch Antikörperbildung erhöht. Die Zufuhr erfolgt im allgemeinen durch subkutane, intramuskuläre oder orale Applikation. Der Wirkstoff muß im allgemeinen in einer sehr hohen Dosis verabreicht werden, um die gewünschte Immunisierung herbeizuführen. Es kann dabei zu Überempfindlichkeitsreaktionen, schlimmstenfalls zu einer Schockreaktion, kommen. Ferner ist zu bedenken, daß bei allen Applikationsarten per Injektion als mögliche Komplikation eine Infektion auftreten kann und daß Applikationen per Injektion nur durch einen Arzt durchgeführt werden dürfen.

Bei oraler Applikation eines Wirkstoffes, wie z.B. Polio-Schluckimpfung, Typhus-Impfung etc., wird ebenfalls eine hohe Konzentration des Wirkstoffes benötigt. Die Aufnahme des Wirkstoffes erfolgt bei dieser Form der oralen Applikationen über die Magen- bzw. Darmschleimhaut. Es kann bei der Verabreichung eines Wirkstoffes zu Überempfindlichkeitsreaktionen (Übelkeit, Erbrechen, Durchfall, Fieber etc.) kommen. Bei der Poliomyelitis (Kinderlähmung), von der insbesondere Kleinkinder, zunehmend aber auch ältere Kinder und Erwachsene, betroffen sind, wird ein Zuckerstück mit dem Wirkstoff versetzt, um die Akzeptanz und Impfbereitschaft zu erhöhen.

Zu den oben beschriebenen von der Applikationsform abhängigen Nachteilen kommen hierbei die Probleme hinzu, die allgemein bei der Immunisierung auftreten. Es ist zum einen eine gesonderte Einnahme des Wirkstoffes nötig, wobei dieser Wirkstoff zwecks dauerhafter Immunisierung in regelmäßigen Abständen erneut eingenommen werden muß. Dies wird häufig vergessen bzw. aus Zeitmangel (Stichwort last-minute-Reisen) nicht durchgeführt, so daß kein ausreichender Impfschutz besteht. Das größte Problem bei der Immunisierung besteht aber zweifellos darin, daß eine große Anzahl an T-Helferzellen generiert werden, die aber häufig wenig affin oder avid bezüglich des Antigens sind. Dadurch wird häufig eine unbefriedigende Immunabwehr erzeugt.

Die Erfindung stellt sich daher die Aufgabe eine Darreichungsform von immunologischen Wirkstoffen bereitzustellen, welche für die spezifische Beeinflussung des Immunsystems geeignet sind. Dabei sollen hochaffine Zellen durch die immunologischen Wirkstoffe angesprochen werden. Ferner sollen die oben beschriebenen Nachteile des Standes der Technik umgangen werden.

Diese Aufgabe wird gelöst durch die Bereitstellung von immunologisch wirksamen Wirkstoffpaaren wie sie in Anspruch 1 beschrieben sind. Bevorzugte Ausführungsformen sind in den Ansprüchen 2 bis 26 dargestellt. Die Ansprüche 27 und 28 betreffen die Verwendung bzw. die Bereitstellung der immunologischen Wirkstoffpaare. Der Wortlaut sämtlicher Ansprüche wird hiermit durch Bezugnahme zum Inhalt der Beschreibung gemacht.

Es ist bekannt, daß viele schwere Erkrankungen dadurch entstehen, daß ihre Erreger hervorragend an den menschlichen Organismus angepaßt sind. Hierzu zählen beispielsweise die intrazellulär wachsenden oder lebenden Erreger wie beispielsweise die Mykobakterien, Chlamydien, die Malariaerreger, aber auch Viren wie zum Beispiel der Hepatitis C-Virus. Viele dieser Erreger sind für den Wirt (Mensch) am Anfang sehr verträglich, da sie in den ersten Wochen bis Monaten wenig zerstörerisch und damit wenig pathogen sind. Das Immunsystem reagiert selbst beim Erkennen von Fremdantigenen nur sehr moderat auf diese. Die Erreger täuschen das Immunsystem bezüglich ihrer Pathogenität und das Immunsystem nimmt an, die Erreger sind harmlos, so wie zum Beispiel aufgenommene Nahrungsmittel, die ja auch bekanntlich Fremdantigene beinhalten. Ohne entsprechende Krankheitssymptome wird keine erfolgreiche Immunantwort induziert. Es ist nun überraschender Weise gefunden worden, daß bei gleichzeitiger Präsentation von sogenannten "Danger-Signalen" das Immunsystem die präsentierten Fremdantigene als Gefahr erkennen und bekämpfen kann.

Erfindungsgemäß sind die immunologischen Wirkstoffe, die für die orale oder nasale Behandlung und insbesondere Aufnahme durch die Mundoder Nasenschleimhaut vorgesehen sind dadurch ausgezeichnet, daß die Wirkstoffe immunologisch wirksame Wirkstoffpaare mit mindestens einem Antigen und mindestens einem Immunsignalstoff sind und zusammen mit mindestens einer Trägersubstanz vorliegen.

In einer bevorzugten Ausführungsform ist ein Immunsignalstoff vorgesehen, der sich dadurch auszeichnet, daß er die durch das Antigen induzierte immunologische Antwort beeinflußt, insbesondere stimuliert oder supprimiert. Als stimulierende Immunsignalstoffe (Danger-Signal) kommen insbesondere solche in Frage, die die T_{H}-1 vermittelte Immunantwort fördern.

Bei den stimulierenden Immunsignalstoffen kann es sich ferner um körpereigene oder analoge Stoffe handeln. Bevorzugt handelt es sich bei den körpereigenen Immunsignalstoffen um solche, die beim Zerfall von Zellen (Nekrose) freigesetzt werden. Bei den stimulierenden körpereigenen Immunsignalstoffen kann es sich um intrazelluläre und/oder extrazelluläre Immunsignalstoffe handeln. Stimulierende körpereigene Immunsignalstoffe werden zum Beispiel beim Zerfall von Leberzellen, Lungenzellen und/oder Erythrozyten freigesetzt. Stimulierende Immunsignalstoffe können zum Beispiel heat-shock-proteins (HSP), TNFalpha, Lektine, Mitogene, Alpha-Interferon, Cytokine und/oder CDs (clusters of differentiation bzw. cluster determinants) sein.

Erfindungsgemäß ist es ferner möglich, das Immunsystem dahingehend zu beeinflussen, daß durch Zugabe eines supprimierenden Immunsignalstoffes die T_{H}-2 vermittelte Immunantwort gefördert wird. Bei diesen supprimierenden Immunsignalstoffen kann es sich insbesondere um solche Signalstoffe handeln, die die IgG und/oder IgA vermittelte Immunantwort fördern. Es ist zum Beispiel möglich, Lebensmittelallergene oder andere Allergene (z.B. Gräserpollen, Hausstauballergene, Birkenpollen, Roggen etc.) in geringen Konzentrationen (abhängig von der Reinheit des Allergens) zusammen mit Immunsignalstoffen zu verabreichen, so daß eine verstärkte IgG- oder lgA-Produktion begünstigt wird. Diese Immunglobuline der IgG- oder lgA-Typ können die allergieverursachenden Immunglobuline vom lgE-Typ verdrängen. Allergien zählen wie auch zum Beispiel Rheuma zu den Autoimmunerkrankungen. Bei der Rheumabehandlung kann man zum Beispiel durch Gabe von Autoantigenen (Kollagen, Heatshockproteine etc.) und gleichzeitiger Verabreichung von supprimierenden Immunsignalstoffen (z.B. Adjuvantien) spezifisch in den Gelenken die T_{H}-2 Zellen aktivieren. Diese hemmen dann die Entzündungsreaktion. Bei den supprimierenden Immunsignalstoffen kann es sich im allgemeinen um körpereigene oder analoge Stoffe handeln. Bei den supprimierenden Immunsignalstoffen kann es sich ferner um Adjuvatien, insbesondere inkomplette Freudsche Adjuvantien, handeln.

Erfindungsgemäß kann sowohl der stimulierende als auch der supprimierende Immunsignalstoff in einer größeren Gewichtsmenge vorliegen als das Antigen. Insbesondere bei einer eventuellen nochmaligen Gabe an Antigen kann die Gewichtsmenge an Immunsignalstoff höher sein als das zu verabreichende Antigen.

Erfindungsgemäß kann es sich bei den Antigenen um immunologisch wirksame Antigene (Immunogene), vorzugsweise um Impfstoffe und/oder Allergene, handeln. So können zum Beispiel Karies- und/oder Parodontoseerreger als Antigene benutzt werden. Das Antigen kann vor Verabreichung ex vivo vermehrt werden. So ist zum Beispiel erfindungsgemäß die Tumorvakzinierung erfaßt. Bei Tumoren gibt es in der Regel ähnliche Probleme bezüglich der Immunantwort wie bei chronischen Infektionen und Autoimmunerkrankungen. Tumore sind im allgemeinen dadurch gekennzeichnet, daß sie im frühen Stadium sehr langsam wachsen und vergleichsweise gut verträglich sind. Das Immunsystem erkennt sie daher nicht als Gefahr für den befallenen Organismus. Eine Tumorvakzinierung bzw. Tumorbehandlung müßte sich zum Ziel machen, Tumorantigene zusammen mit Danger-Signalen zu präsentieren. So ist es zum Beispiel möglich, nach operativer Entfernung des Tumors oder Teilen davon dessen Antigene aus dem Tumorgewebe zu isolieren. Insbesondere solche Stoffe sind für eine Antigenpräsentation geeignet, die sich an den Heatshockproteinen gefaltet haben, da sie tumorspezifisch oder zumindestens tumorassoziiert sind. Diese Antigene bzw. auch eventuell zu präparierendes Tumorantigenextrakt können zusammen mit einem oder mehreren verschiedenen Dangersignalen präsentiert werden. Als Danger-Signale eignen sich insbesondere Adjuvantien, Mitogene, körpereigene Warnsignale wie alpha-Interferon aber auch zytoplasmatische Antigene wie Heat-shock-Proteine. Alternativ oder in Kombination können die Antigene ex vivo vermehrt und dem Organismus erneut zugeführt werden.

Erfindungsgemäß kann es ferner vorgesehen sein, daß das Antigen zur mindestens zweimaligen Verabreichung vorgesehen ist. Als Antigen im Sinne der Erfindung können Peptide und/oder Polypeptide genutzt werden, wobei diese Peptide und/oder Polypeptide mindestens sieben Aminosäuren lang sind, damit eine immunologische Antwort erfolgen kann. Ferner sind als Antigene auch Polynukleotide, insbesondere DNA und/oder RNA geeignet, wobei die Polynukleotide selbst aber auch das von ihnen dekodierte Produkt als Antigen dienen kann. Das Antigen kann in einer Dosis vorliegen, die 10 % bis 50 % der Menge entspricht, in der das gleiche Antigen normalerweise für die subkutane oder intramuskuläre Verabreichung vorliegen würde, wenn kein Immunsignalstoff mithinzugegeben wird.

Bei der erfindungsgemäßen Darreichungsform können im allgemeinen niedrigere Konzentrationen an einem Antigen verabreicht werden. Dadurch werden die hoch affinen oder aviden T-Helferzellen generiert. Die anschließende klonale Selektion wird sich daher auf die antigenspezifischen Zellen beschränken, die am effektivsten das in niedriger Konzentration präsentierte Antigen oder damit auch den Erreger erkennen und auch bekämpfen können. Durch diese Form der Verabreichung wird vermieden, daß viele verschieden T-Helferzellen aktiviert werden, die zum Beispiel zwar den Erreger oder ein Epitop des Erregers erkennen können, gegen diesen aber nur wenig effektiv sind. In diesem Zusammenhang bedeutet weniger Effektivität eine geringere Affinität und/oder geringere Aktivierbarkeit, die sich beispielsweise anhand der Citokinausschüttung messen läßt. So ist es zum Beispiel möglich, bei der Bekämpfung des Hepatitis C-Virus folgende Strategie anzuwenden. Um hoch affine T-Helferzellen zu generieren können als Antigene Peptide aus der NS 1, 2 oder 3-Region bzw. andere Peptide, die ein Epitop des Hepatitis-Virus darstellen, ausgewählt und dann in geringeren Konzentrationen als üblich zusammen mit einem weiteren Immunsignalstoff verabreicht werden, der dann als "Danger-Signal" fungieren kann.

"Danger-Signal" in diesem Zusammenhang können insbesondere verschiedene Stoffe (z.B. Proteine) sein, die beim Zerfall von Leberzellen (nekrotischer Zerfall) freigesetzt werden. Dem Körper kann dadurch vermittelt werden, daß das Antigen eine Gefahr darstellt, da gleichzeitig mit dessen Auftreten auch intrazelluläre Zerfallsprodukte präsentiert werden. Durch die Kopplung von Antigenen mit Zerfallsprodukten von Zellen kann dem Immunsystem suggeriert werden, daß die Antigene für den Zerfall der Zellen verantwortlich sind. Das Immunsystem kann daher mit dem Generieren von T-Zellen antworten, die spezifisch die präsentierten Epitope, zum Beispiel die Hepatitis C-Epitope, erkennen und bekämpfen. Ein weiteres Danger-Signal kann auch zum Beispiel alpha-Interferon sein. Alternativ oder in Kombination kann dann in einem weiteren Schritt die Vermehrung der hochaffinen Zellen erfolgen. Dies kann erfindungsgemäß beispielsweise durch die nochmalige Gabe größerer Konzentrationen an gleichen Antigenen und/oder Danger-Signal(en) erfolgen. Die spezifischen T-Helferzellen werden dadurch stark vermehrt, wodurch eine effektive Immunreaktion induziert werden kann. Bei der Behandlung von Mykobakterien ist es zum Beispiel möglich, verschiedene mykobakterienspezifische Peptide und/oder Proteine oder Mischungen aus Peptiden und/oder Proteinen sowie auch andere Substanzen, die als mykobakterienspezifische Antigene fungieren können, gemeinsam mit einem Danger-Signal zu verabreichen. Danger-Signale bezüglich Mykobakterienbefall können zum Beispiel verschiedene zytoplasmatische oder zelluläre Strukturen aus den Lungen sein. Weitere Danger-Signale können zum Beispiel Heat-shock-Proteine (HSP) oder auch alpha-Interferon sein. Durch die gleichzeitige Verabreichung von spezifischen Antigenen in geringen Konzentrationen und einem oder mehreren Danger-Signalen können hochaffine T-Helferzellen generiert werden, die in einem weiteren Schritt vermehrt werden können. Eine ähnliche Strategie kann zum Beispiel auch bei der Bekämpfung des Malariaerregers genutzt werden. Hierbei können spezifische Malariaantigene zusammen mit Danger-Signalen verabreicht werden wie zum Beispiel Danger-Signale der Leber oder der Erythrozyten, insbesondere Leberzellproteine oder Erythrozytenproteine. Diese Strategie kann sowohl zur Behandlung als auch zur Prophylaxe von Infektionen genutzt werden.

Die erfindungsgemäße Darreichungsform ist für die orale oder nasale Behandlung und insbesondere Aufnahme durch die Mund- oder Nasenschleimhaut vorgesehen in Form der immunologischen Wirkstoffpaare zusammen mit mindestens einer Trägersubstanz. Als Trägersubstanz können insbesondere die Substanzen dienen, die ohnehin regelmäßig, insbesondere täglich ein- und/oder aufgenommen werden. Als Trägersubstanz können ferner Stoffe genutzt werden, die eine hohe Verweildauer im Mund, vorzugsweise von 20 Sekunden bis 1 Stunde aufweisen. Bei der Trägersubstanz kann es sich um alltägliche Stoffe, wie zum Beispiel Mundhygieneartikel oder Süßwaren, handeln. So ist es zum Beispiel möglich, die immunologisch wirksamen Wirkstoffpaare einer Zahnpasta, einem Mundwasser, Mundspülungen, Schokolade, Bonbons, Kaugummi etc., bei nasaler Verabreichung einem Nasenspray, beizumengen. Bei der Prophylaxe bzw. Behandlung von Karies oder Parodontosis ist es zum Beispiel erfindungsgemäß vorgesehen, spezifische Antigene zusammen mit Danger-Signalen (zum Beispiel Heat-shock-Proteine) der Zahnpasta beizumengen. Durch die regelmäßige Aufnahme dieser Wirkstoffpaare bzw. der direkten Wirkung im Mund ist eine Prophylaxe bzw. Behandlung gegeben. Allgemein ist in diesem Zusammenhang zu erwähnen, daß durch die Aufnahme über die Mund- bzw. Nasenschleimhaut die immunologische Antwort schon zu einem sehr frühen Zeitpunkt erfolgen kann, ohne daß das Antigen durch den Magen-Darm-Trakt transportiert werden muß. Bei der Trägersubstanz kann es sich ferner auch um eine solche handeln, die normalerweise nicht zur Ernährung dient, die insbesondere auch nicht abgeschluckt wird.

Erfindungsgemäß kann die Beimengung der Wirkstoffpaare in die Trägersubstanz vorgegeben sein, diese Beimengung kann aber auch dadurch erfolgen, daß die Beimengung von mindestens einem Wirkstoff durch den Konsumenten erfolgt.

In einer bevorzugten Ausführungsform werden als Wirkstoffe mindestens zum Teil Naturstoffe vorgegeben, insbesondere solche Naturstoffe, die lediglich angereichert sind. Diese Naturstoffe können als Stimulanzien zur Stärkung des Immunsystems gegen Allergene und körpereigene Bakterien dienen. Als Naturstoffe, d.h. als natürliche Stimulanzien, wären zum Beispiel die Sojabohnenpräparate zu nennen, die zusammen mit Birkenund/oder Haselnuswirkstoffen (als Allergene) verabreicht werden. Diese Wirkstoffe können mindestens zum Teil pflanzlichen Ursprunges sein.

Die Erfindung umfaßt auch Verfahren zur Herstellung einer Darreichungsform von immunologischen Wirkstoffen für die orale oder nasale Behandlung und insbesondere Aufnahme durch die Mund- oder Nasenschleimhaut, wobei diese Darreichungsform durch die weiter oben beschriebenen Merkmale gekennzeichnet ist.

Schließlich wird die Bereitstellung einer Darreichungsform nach einem der oben beschriebenen Merkmale zur Stimulierung oder Supprimierung des Immunsystems beansprucht.

Weitere Einzelheiten der Erfindung ergeben sich aus der Beschreibung der nachfolgenden Beispiele in Verbindung mit den Unteransprüchen. Hierbei können die Merkmale jeweils für sich oder in Kombination miteinander verwirklicht sein.

### BEISPIELE

### Beispiel 1: Immunstimulation

Karies- und/oder Paradontoseerreger werden einer handelsüblichen Zahncreme in einer Menge von kleiner 0,1 Gew.% pro 100 Gew.% Zahncreme beigegeben. Neben dieser antigenen Determinante werden der gleichen Zahncreme Heat-shock-Proteine (HSP) in einer Menge von ca. 0,1 Gew.% pro 100 Gew.% Zahncreme beigemengt. Ein männlicher Probant mit reversiblem Frühstadium der Zahnkaries mit teilweiser (poröser) Entkalkung des kristallinen Zahnschmelzes wurde über einen Zeitraum von einigen Monaten zweimal täglich mit der Zahncreme behandelt. Es trat eine deutliche Verbesserung der Zahnkaries auf, mit teilweiser Wiederverkalkung (Remineralisierung). Bei einem weiteren Probanten wurden die Karieserreger sowie die Heat-Shock-Proteine einem handelsüblichen Mundwasserpräparat beigegeben. Auch bei diesem Probanten zeigte sich nach einigen Monaten eine drastische Verbesserung der Zahnkaries. Es wurde eine Wiederverkalkung (Remineralisierung) diagnostiziert. Die gleichen Versuche wurden mit Sojabohnen- und Mistelpräparaten als stimulierende Immunsignalstoffe durchgeführt, und es wurden ähnliche Resultate erzielt wie bei den HSP-Versuchen.

### Beispiel 2: Allergene

Lebensmittelallergene werden einem Kaugummi beigegeben. Als supprimierender Immunsignalstoff wird inkomplette Freudsche Adjuvanz dem Kaugummi hinzugefügt. Eine weibliche Probantin mit einer Lebensmittelallergie wird über einen Zeitraum von 6 Monaten mit diesem Kaugummi behandelt. Nach dieser 6-monatigen Behandlung konnte im Blut der Patientin ein erhöhter IgG-Spiegel sowie eine erhöhte Antigen spezifische T_{H}-2-Zellzahl nachgewiesen werden. Es erfolgte eine Desensibilisierung bezüglich der Lebensmittelallergene. Nachteilige Wirkungen sowohl subjektiv als auch objektiv wurden während des experimentellen Zeitraumes bei der Patientin nicht beobachtet.

### Beispiel 3: Vakzinierung

a) Hepatitis-C-Vakzinierung:
   Als Antigene wurden Peptide aus der NS 1, 2 und 3 Region einem Bonbon beigegeben. Zusammen mit diesen Antigenen wurden als stimulierende Immunsignalstoffe alpha-Interferon dem gleichen Bonbon beigefügt. Über einen Zeitraum von einigen Monaten wurde einem Probanten, der zuvor Hepatitis-C negativ klassifiziert wurde, zweimal täglich dieses Bonbon zur oralen Behandlung verabreicht. Nach einigen Monaten fand eine weitere Boosterung statt, so daß die Grundimmunisierung abgeschlossen wurde. Danach wurde die Serumkonzentration von Anti-HCs-Ag bestimmt, wobei es sich zeigte, daß die Serumkonzentration an Anti-HCs-Ag bei dem Probanten erhöht war.
b) Mykobakterien-Vakzinierung:
   Eine Mischung von BCG (Bacille-Calmette-Guèrin) mit einzelnen mykobakterienspezifischen Antigenen wurden einem Kaugummi beigegeben. Als stimulierender Immunsignalstoff wurde eine Kombination aus Heat-shock-Proteinen und alpha-Interferon dem obigen Kaugummi beigegeben. Ein Probant wurde über einen Zeitraum von einigen Monaten zweimal täglich für mehrere Min. der Kaugummi zur Behandlung verabreicht. Nach einigen Monaten wurde die Serumkonzentration von Anti-TBC-Ag bestimmt, wobei sich eine erhöhte Serumkonzentration nachweisen ließ.
c) Malaria-Vakzinierung:
   Verschiedene Antigene der einzelnen Malariaerreger werden zusammen mit Leberzell- und/oder Erythrozytenproteinen verabreicht. Im Serum wird dann die Konzentration an Antikörpern bestimmt, wobei diese immer nach der Immunisierung erhöht war.
d) Tumor-Vakzinierung:
   Einem Probanten wurde ein Tumor operativ entfernt. Antigene aus diesem Tumorgewebe wurden isoliert, wobei insbesondere die Antigene ausgewählt wurden, die sich zuvor an den Heat-shock-Proteinen gefaltet haben. Diese Tumorantigene wurden zusammen mit verschiedenen stimulierenden Immunsignalstoffen ("Danger-Signalen", alpha-Interferon, Heat-shock-Proteine) dem Probanten oral gegeben. Es zeigte sich, daß eine erhöhte Immunstimulierung nachgewiesen werden konnte, und daß das Allgemeinbefinden des Patienten sich spürbar verbesserte.

## Patentansprüche

1. Darreichungsform von immunologischen Wirkstoffen für die orale oder nasale Behandlung und insbesondere Aufnahme durch die Mund- oder Nasenschleimhaut, **dadurch gekennzeichnet, daß** die Wirkstoffe immunologisch wirksame Wirkstoffpaare mit mindestens einem Antigen und mindestens einem Immunsignalstoff sind und zusammen mit mindestens einer Trägersubstanz vorliegen.

2. Darreichungsform nach Anspruch 1, **dadurch gekennzeichnet, daß** mindestens ein Immunsignalstoff vorgesehen ist, der die durch das Antigen induzierte immunologische Antwort beeinflußt, insbesondere stimuliert oder supprimiert.

3. Darreichungsform nach Anspruch 2, **dadurch gekennzeichnet, daß** es sich bei den stimulierenden Immunsignalstoffen um solche handelt, die die T_{H}-1 vermittelte Immunantwort fördern.

4. Darreichungsform nach einem der Ansprüche 2 oder 3, **dadurch gekennzeichnet, daß** es sich bei den stimulierenden Immunsignalstoffen um körpereigene oder analoge Stoffe handelt, wobei insbsondere es sich bei den stimulierenden körpereigenen Immunsignalstoffen um solche handelt, die beim Zerfall von Zellen (Nekrose) freigesetzt werden, und/oder es sich bei den stimulierenden körpereigenen Immunsignalstoffen um intrazelluläre und/oder zelluläre Immunsignalstoffe handelt, und/oder es sich bei den stimulierenden körpereigenen Immunsignalstoffen um solche handelt, die beim Zerfall von Leberzellen, Lungenzellen und/oder Erythrocyten freigesetzt werden.

5. Darreichungsform nach einem der Ansprüche 2 bis 4, **dadurch gekennzeichnet, daß** es sich bei den stimulierenden Immunsignalstoffen um HSP, TNF-alpha, Lektine, Mitogene, alpha Interferon, Cytokine und/oder CDs handelt.

6. Darreichungsform nach Anspruch 2, **dadurch gekennzeichnet, daß** es sich bei den supprimierenden Immunsignalstoffen um solche handelt, die die T_{H}-2 vermittelte Immunantwort fördern, und/oder es sich bei den supprimierenden Immunsignalstoffen um solche handelt, die die lgG und/oder IgA vermittelte Immunantwort fördern, und/oder es sich bei den supprimierenden Immunsignalstoffen um körpereigene oder analoge Stoffe handelt, und/oder es sich bei den supprimierenden Immunsignalstoffen um Adjuvantien, insbesondere um inkomplette Freudsche Adjuvantien, handelt.

7. Darreichungsform nach einem der vorhergehenden Ansprüche, **dadurch gekennzeichnet, daß** der Immunsignalstoff in einer größeren Gewichtsmenge vorliegt als das Antigen.

8. Darreichungsform nach einem der vorhergehenden Ansprüche, **dadurch gekennzeichnet, daß** es sich bei dem Antigen um immunologisch wirksame Antigene (Immunogen), vorzugsweise Impfstoffe und/oder Allergene, handelt, wobei insbesondere das Antigen in ex vivo vermehrter Form vorliegt, und/oder das Antigen zur mindestens zweimaligen Verabreichung vorgesehen ist, und/oder es sich bei dem Antigen um ein Peptid und/oder Polypeptid handelt, wobei dieses Peptid und/oder Polypeptid mindestens sieben Aminosäuren lang ist, oder es sich bei dem Antigen um ein Polynukleotid, insbesondere DNA und/oder RNA handelt, und dieses Polynukleotid das Antigen dekodiert, und/oder das Antigen in einer Dosis vorliegt, die 10% bis 50% der Menge entspricht, in der das gleiche Antigen normalerweise für die subkutane oder intramuskuläre Verabreichung vorliegt.

9. Darreichungsform nach einem der vorhergehenden Ansprüche, **dadurch gekennzeichnet, daß** die Trägersubstanz eine solche ist, die ohnehin regelmäßig, insbesondere täglich, ein- und/oder aufgenommen wird, wobei insbesondere als Trägersubstanz Stoffe mit einer hohen Verweildauer im Mund, vorzugsweise von 20 Sekunden bis 1 Stunde, ausgewählt werden, und/oder es sich bei der Trägersubstanz um alltägliche Stoffe, vorzugsweise Mundhygieneartikel oder Süßwaren, handelt, und/oder es sich bei der Trägersubstanz um eine solche handelt, die normalerweise nicht zur Ernährung dient, insbesondere nicht geschluckt wird.

10. Darreichungsform nach einem der vorhergehenden Ansprüche, **dadurch gekennzeichnet, daß** die Beimengung von mindestens einem Wirkstoff in der Trägersubstanz vorgegeben ist und/oder durch den Konsumenten erfolgt.

11. Darreichungsform nach einem der vorhergehenden Ansprüche, **dadurch gekennzeichnet, daß** als Wirkstoffe mindestens zum Teil Naturstoffe vorgesehen sind, insbesondere solche, die lediglich angereichert sind.

12. Darreichungsform nach einem der vorhergehenden Ansprüche, **dadurch gekennzeichnet, daß** die Wirkstoffe mindestens zum Teil pflanzlichen Ursprungs sind.

13. Verfahren zur Herstellung einer Darreichungsform von immunologischen Wirkstoffen für die orale oder nasale Behandlung und insbesondere Aufnahme durch die Mund- oder Nasenschleimhaut, **gekennzeichnet durch** die Merkmale des kennzeichnenden Teils einer der vorhergehenden Ansprüche 1 bis 12.

14. Bereitstellung einer Darreichungsform nach einem der Ansprüche 1 bis 12, zur Stimulierung oder Supprimierung des Immunsystems.
